# EUROPEAN PATENT APPLICATION

(11) **EP 3 939 631 A1**
(43) Date of publication of application: **19.01.2022**
(21) Application number: 20769698.0
(22) Date of filing: 07.02.2020
(51) Int. Cl.: A61M 1/02, A61J 1/05

(54) **BLOOD BAG SYSTEM**

(30) Priority: 14.03.2019 JP 2019047060
(71) Applicant: TERUMO Kabushiki Kaisha, Tokyo 151-0072 (JP)
(72) Inventor: AGEISHI, Aya, Tokyo 163-1450 (JP); MARUTA, Chiaki, Tokyo 163-1450 (JP)
(74) Representative: TBK
(86) International application number: PCT/JP2020/004797
(87) International publication number: WO 2020/184019

(57) **Abstract**

A blood bag system (10) is provided with a PPP bag (24) that is accommodated in a PPP bag pocket (58), and a liquid drug bag (26) that is accommodated in a liquid drug bag pocket (60) provided adjacent to the PPP bag pocket (58). The PPP bag (24) includes a first internal space (24a) and a seal part (90) that seals the periphery of the first internal space (24a). The liquid drug bag (26) includes a second internal space (26a) and a seal part (102) that seals the periphery of the second internal space (26a). A connection part (110) that separably connects the seal part (90) and the seal part (102) is provided therebetween.

## Description

### Technical Field

The present invention relates to a blood bag system set in a centrifugal transport device.

### Background Art

A blood bag system including a blood bag storing blood, a PPP bag storing platelet poor plasma, which is a blood component, a liquid drug bag storing a red blood cell preservation solution, and the like is set in a centrifugal transport device at the time of centrifuging blood (see JP H5-184667 A). The centrifugal transport device applies a centrifugal force to the set blood bag to separate the blood in the blood bag into a plurality of types of blood components, and transports a plasma component to the PPP bag via a tube connected to the blood bag. After taking out the blood bag system from the centrifugal transport device, the liquid drug in the liquid drug bag is transported to the blood bag in which concentrated red blood cells remain.

### Summary of Invention

Meanwhile, when the blood bag system is set, the PPP bag is accommodated in a PPP bag pocket (bag accommodating portion) of the centrifugal transport device, and the liquid drug bag is accommodated in a liquid drug bag pocket (bag accommodating portion) of the centrifugal transport device. At this time, a tube connected to the PPP bag or the liquid drug bag is also accommodated in a predetermined path of the centrifugal transport device or the liquid drug bag pocket. If the tube projects, the tube is likely to interfere with a peripheral structure of the centrifugal transport device and break during centrifugation.

However, the work of accommodating the PPP bag and the liquid drug bag in the respective pockets while disposing the tube in the predetermined path requires a large amount of time and effort for a user, and there is a problem that it takes time to set the blood bag system with respect to the centrifugal transport device.

The present invention has been made to solve the above problem, and an object thereof is to provide a blood bag system capable of significantly improving working efficiency by facilitating work of disposing a plurality of bags in a centrifugal transport device with a simple configuration.

In order to achieve the above object, a first aspect of the present invention is a blood bag system including a bag body which includes a plurality of bags accommodated in a bag accommodating portion of a centrifugal transport device. The bag body includes: a first bag including a first internal space and a first seal part that seals a periphery of the first internal space; and a second bag including a second internal space and a second seal part that seals a periphery of the second internal space. A connection part that separably connects the first seal part and the second seal part is provided therebetween.

In order to achieve the above object, a second aspect of the present invention is a blood bag system molded into a bag shape by overlapping sheets made of a resin material and sealing the periphery thereof. The blood bag system includes: a first bag section including a first internal space and a first seal part that seals a periphery of the first internal space; a second bag section including a second internal space and a second seal part that seals a periphery of the second internal space; and a connection part that separates the first bag section and the second bag section and connects the first bag section and the second bag section to be separable as an independent bag.

The above blood bag system includes the connection part that separably connects the first seal part and the second seal part, so that the first bag (first bag section) and the second bag (second bag section) can be integrally handled. That is, a user can smoothly perform the work of disposing the first bag and the second bag in the bag accommodating portion of the centrifugal transport device by collectively gripping the first bag and the second bag. Further, the blood bag system can separate the first bag and the second bag at the connection part when the blood bag system is taken out from the centrifugal transport device, and thus, the subsequent work is speeded up. Therefore, the blood bag system can significantly improve the work efficiency.

### Brief Description of Drawings

Fig. 1 is an explanatory view illustrating an overall configuration of a blood bag system and a centrifugal transport device according to a first embodiment of the present invention.
Fig. 2 is a plan view illustrating a unit set area of the centrifugal transport device.
Fig. 3 is an enlarged plan view illustrating a PPP bag, a liquid drug bag, and a connection part.
Fig. 4 is an explanatory view illustrating an operation at the time of setting the PPP bag and the liquid drug bag.
Fig. 5A is an enlarged plan view illustrating a PPP bag, a liquid drug bag, and a connection part according to a second embodiment of the present invention. Fig. 5B is an enlarged perspective view of a connection member.
Fig. 6 is an enlarged plan view illustrating a PPP bag, a liquid drug bag, and a connection part according to a third embodiment of the present invention.

### Description of Embodiments

Hereinafter, preferred embodiments of the present invention will be described in detail with reference to the accompanying drawings.

### [First Embodiment]

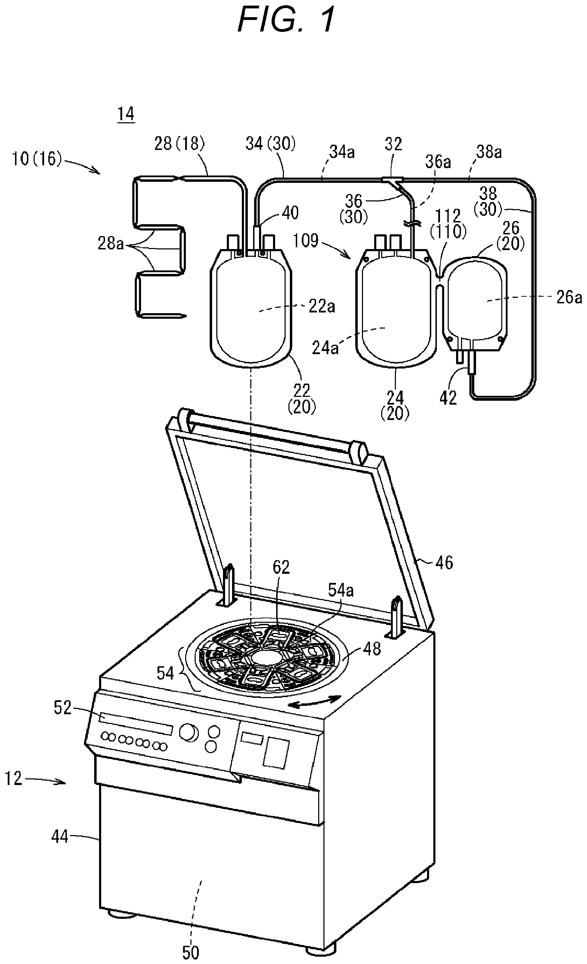

A blood bag system 10 according to a first embodiment of the present invention is set in a centrifugal transport device 12 as illustrated in Fig. 1 by a user such as a medical worker. The centrifugal transport device 12 centrifuges blood in the blood bag system 10 to generate and store a plurality of types of blood components. Hereinafter, a configuration including the blood bag system 10 and the centrifugal transport device 12 is referred to as a blood product manufacturing system 14.

The blood bag system 10 includes a pretreatment unit (not illustrated) used for blood sampling before centrifugation and a separator unit 16 that generates blood components by centrifugation and individually stores the blood components, and the both units are connected by a relay tube 18. The separator unit 16 is set in the centrifugal transport device 12 in a state where the relay tube 18 connected to the pretreatment unit is cut into the state illustrated in Fig. 1 before the centrifugation.

The pretreatment unit of the blood bag system 10 collects whole blood from a donor (blood donor) and removes a predetermined blood component (for example, a white blood cell) from the whole blood. Therefore, the pretreatment unit includes a blood collection needle, a blood collection bag, an initial flow blood bag, a filter (for example, a white blood cell removal filter), and the like, and is configured by connecting the respective members via a plurality of tubes. The separator unit 16 is connected to the downstream side of the filter via the relay tube 18. Specifically, the pretreatment unit stores the first blood out of the whole blood of the donor, collected through the blood collection needle, in the initial flow blood bag, and then stores the remaining blood in the blood collection bag. Further, the pretreatment unit removes white blood cells in the filter by causing the whole blood, stored in the blood collection bag, to flow to the filter, and transports the removed blood (white-blood-cell-removed blood) to the separator unit 16.

On the other hand, the separator unit 16 of the blood bag system 10 includes a plurality of bags 20 (a blood bag 22, a PPP bag 24, and a liquid drug bag 26), and is configured by connecting the respective bags 20 via a plurality of tubes 30.

The blood bag 22 is directly connected to the relay tube 18 connected to the pretreatment unit in a state of the blood bag system 10 provided as a product. Therefore, the blood bag 22 stores the removed blood transported from the filter at the time of collecting blood. The blood bag 22 is set in the centrifugal transport device 12, and a centrifugal force is applied by the operation of the centrifugal transport device 12. As a result, the removed blood in the blood bag 22 is centrifuged into blood components (platelet poor plasma (PPP), red blood cells (RBC)) and the like having different specific gravities. Then, the blood bag 22 transports PPP under the operation of the centrifugal transport device 12 after centrifugation to store only the remaining RBC.

The PPP bag 24 is a medical bag (plasma bag: first bag) that stores PPP (plasma component) in a storage space 24a by receiving the PPP supplied from the blood bag 22. On the other hand, the liquid drug bag 26 is a medical bag (second bag) in which a red blood cell preservation solution (liquid drug), such as a MAP solution, an SAGM solution, and OPTISOL, is stored in advance in a storage space 26a.

In addition, a segment tube 28 is formed before the separator unit 16 is set in the centrifugal transport device 12. The segment tube 28 has a plurality of sealed tubes 28a in which blood of the donor (removed blood) exists. The plurality of sealed tubes 28a are formed so as to be continuous in series by cutting the relay tube 18, which connects the blood bag 22 and the pretreatment unit (filter), near the filter and further sealing the cut tube at predetermined intervals. The segment tube 28 is cut by a user as necessary, and is used for checking blood or the like.

The plurality of tubes 30 of the separator unit 16 branch into a plurality of tubes via a branching connector 32 (Y-type connector). Specifically, the plurality of tubes 30 include a first tube 34 connecting the blood bag 22 and the branching connector 32, a second tube 36 (first tubular body) connecting the PPP bag 24 and the branching connector 32, and a third tube 38 (second tubular body) connecting the liquid drug bag 26 and the branching connector 32. The first tube 34 has a first flow path 34a, the second tube 36 has a second flow path 36a, and the third tube 38 has a third flow path 38a. The first to third flow paths 34a, 36a, and 38a communicate with each other via a flow path in the branching connector 32.

A breakable sealing member 40 (click tip) is provided at an end portion of the first tube 34 on the blood bag 22 side. Similarly, a breakable sealing member 42 is provided at an end portion of the third tube 38 on the liquid drug bag 26 side. The sealing members 40 and 42 block the first flow path 34a and the third flow path 38a until a breaking operation is performed, and prevent a liquid in the blood bag 22 and the liquid drug bag 26 from being transported to the other bag 20.

On the other hand, the centrifugal transport device 12 in which the separator unit 16 of the blood bag system 10 is set includes a box-shaped base body 44, a lid 46 capable of opening and closing an upper surface of the base body 44, and a centrifugal drum 48 provided on the base body 44. In addition, the base body 44 of the centrifugal transport device 12 is provided with a motor (not illustrated) that rotates the centrifugal drum 48, a control unit 50 that controls the operation of the centrifugal transport device 12, and an operation display unit 52 configured to allow the user to perform confirmation and operation.

The centrifugal drum 48 has a plurality of (six) unit set areas 54 in which the separator unit 16 can be set. A height of one unit set area 54 is longer than a longitudinal length of the bag 20, and is set in a range of 60° with respect to a rotation center of the centrifugal drum 48. That is, the six unit set areas 54 are arrayed without a gap along the circumferential direction to form the centrifugal drum 48 which is an annular structure.

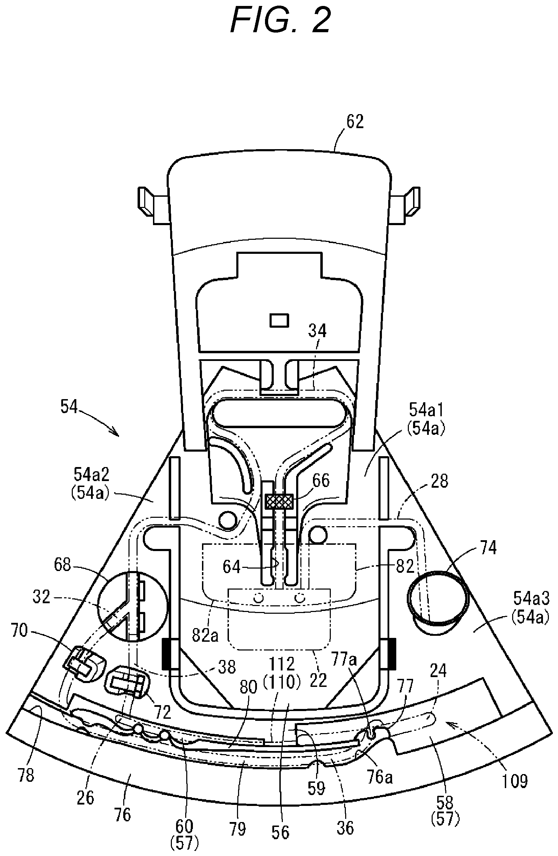

As illustrated in Figs. 1 and 2, the unit set area 54 applies the centrifugal force to the blood bag 22 as the centrifugal drum 48 rotates. Specifically, the unit set area 54 includes a blood bag pocket 56 accommodating the blood bag 22, a PPP bag pocket 58 (first bag accommodating portion) accommodating a PPP bag 24, and a liquid drug bag pocket 60 (second bag accommodating portion) accommodating the liquid drug bag 26 at radially outer positions of the centrifugal drum 48.

The blood bag pocket 56 is provided at the circumferential center of the unit set area 54 and has a larger volume than the PPP bag pocket 58 or the liquid drug bag pocket 60. The PPP bag pocket 58 and the liquid drug bag pocket 60 are bag accommodating portions 57 provided side by side (to be adjacent) in the circumferential direction on the radially outer side of the blood bag pocket 56. A boundary wall 59 extending in the radial direction between the upper surface 54a of the unit set area 54 and an outer peripheral edge of the centrifugal drum 48 is provided between the PPP bag pocket 58 and the liquid drug bag pocket 60.

In addition, an upper surface 54a of the unit set area 54 is configured such that the plurality of tubes 30 of the blood bag system 10 are disposed and retained thereon. The first tube 34 and a part of the segment tube 28 are disposed in a central region 54a1 (first region) on the radially inner side of the blood bag pocket 56 on the upper surface 54a. The central region 54a1 is provided with a lid body 62 that opens and closes an opening of the blood bag pocket 56. Further, a sensor 66 (optical sensor), which detects a part of a breaking portion 64 that breaks the sealing member 40 and a state of blood flowing through the first tube 34, is provided at an arrangement position of the first tube 34 closed by the lid body 62.

A part of the first tube 34 passing through the central region 54a1, the branching connector 32, a part of the second tube 36, and a part of the third tube 38 are disposed in a left region 54a2 (second region) of the upper surface 54a. The left region 54a2 is provided with a holder 68 that retains the branching connector 32, a PPP clamp 70 that opens and closes the second flow path 36a of the second tube 36, and a liquid drug clamp 72 that opens and closes the third flow path 38a of the third tube 38.

A part of the segment tube 28 passing through the central region 54a1 is disposed in a right region 54a3 (third region) of the upper surface 54a. The right region 54a3 is provided with a segment pocket 74 that accommodates the plurality of sealed tubes 28a of the segment tube 28.

Further, a tube retaining portion 76 protruding upward from the upper surface 54a is provided on the radially outer side of the PPP bag pocket 58 and the liquid drug bag pocket 60 of the unit set area 54. The tube retaining portion 76 has an outer wall 78 continuous with an outer peripheral edge of the unit set area 54 and an inner wall 80 protruding to the inner side of the outer wall 78 from the outer peripheral edge, and forms a guide groove 79 for disposing the second tube 36 between the outer wall 78 and the inner wall 80. The inner wall 80 extends from the vicinity of the PPP clamp 70 in the left region 54a2 (left side of the liquid drug bag pocket 60) to a circumferential intermediate position of the PPP bag pocket 58, and defines an extending length of the guide groove 79.

In addition, one end portion of the tube retaining portion 76 overlapping the PPP bag pocket 58 forms a one-end-side inclined surface 76a that protrudes so as to be inclined to the inner side in the centrifugal direction (radial direction) from the circumferential direction along the outer peripheral edge. The one-end-side inclined surface 76a is provided with a locking plate 77 protruding to the inner side in the centrifugal direction. A notch 77a capable of locking the second tube 36 is formed in the locking plate 77.

In addition, the unit set area 54 includes a slider 82 (pusher), which presses the blood bag 22 after centrifugation, on the radially inner side of the blood bag pocket 56. A surface of the slider 82 facing the blood bag 22 is formed on an inclined surface 82a. The slider 82 moves forward and backward along the radial direction of the centrifugal drum 48 under the control of the control unit 50 (see Fig. 1).

In the unit set area 54, the user accommodates the blood bag 22 storing the removed blood, the empty PPP bag 24, and the liquid drug bag 26 storing the liquid drug in the blood bag pocket 56, the PPP bag pocket 58, and the liquid drug bag pocket 60, respectively. Then, the unit set area 54 centrifuges the removed blood in the blood bag 22 by the rotation of the centrifugal drum 48, advances the slider 82 after the centrifugation to press the blood bag 22. As a result, PPP generated by the centrifugation in the blood bag 22 is transported to the PPP bag 24, and RBC remains in the blood bag 22 after the transport of PPP. Thereafter, the blood bag system 10 is taken out from the centrifugal transport device 12 by the user, and the liquid drug bag 26 is hung on a stand (not illustrated). As a result, the red blood cell preservation solution is supplied from the liquid drug bag 26 to the blood bag 22, and the blood bag 22 is turned into a state of storing the RBC (blood products) containing a liquid drug.

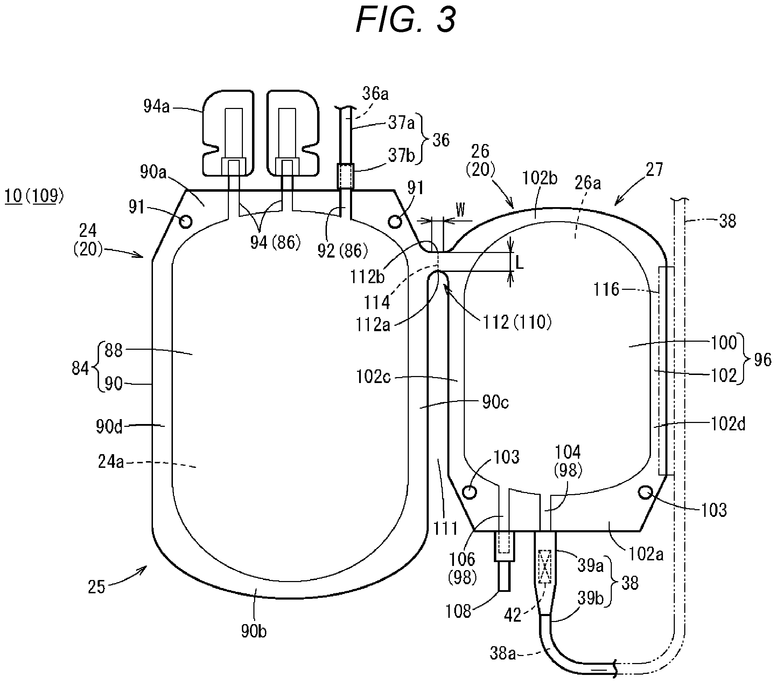

Next, the PPP bag 24, the liquid drug bag 26, and the like of the blood bag system 10 according to the present embodiment will be described with reference to Fig. 3.

The PPP bag 24 includes a bag main body 84 having the storage space 24a (first internal space) capable of storing PPP, and a plurality of ports 86 provided on one side of the bag main body 84. The bag main body 84 is molded in a bag shape by overlapping sheets to seal the periphery.

Specifically, the bag main body 84 is formed in a substantially rectangular shape in plan view, and includes an accommodating portion 88 forming the storage space 24a and a seal part 90 (first seal part) which is a portion sealed around the accommodating portion 88. A resin material forming the bag main body 84 is not particularly limited, and examples thereof include polyolefins such as polyvinyl chloride, an ethylene-vinyl acetate copolymer, polyethylene, and polypropylene.

The seal part 90 includes one short side seal 90a of the bag main body 84, the other short side seal 90b of the bag main body 84, one long side seal 90c of the bag main body 84, and the other long side seal 90d of the bag main body 84 in plan view. In Fig. 3, the one short side seal 90a, the other short side seal 90b, the one long side seal 90c, and the other long side seal 90d are located on the upper side, the lower side, the right side, and the left side, respectively. In addition, two (one set of) bag holes 91 are provided at corner portions of the one and other long side seals 90c and 90d continuous with the one short side seal 90a.

The plurality of ports 86 are provided on the one short side seal 90a of the bag main body 84. The plurality of ports 86 include a connection port 92 to which the second tube 36 is connected, and two extraction ports 94 covered with a cover 94a that blocks outflow of blood. The plurality of ports 86 are welded in the state of communicating with the storage space 24a at the time of forming the seal part 90. The connection port 92 is firmly fixed to an end portion of the second tube 36 by an appropriate fixing means such as welding and adhesion.

The second tube 36 includes an elongated tube main body 37a forming most of its entire length, and an end structure 37b that is continuous with an end portion of the tube main body 37a and is formed thick to be connected to the connection port 92.

Examples of a material forming the second tube 36 include a fluorine-based resin such as polytetrafluoroethylene (PTFE), an ethylene-tetrafluoroethylene copolymer (ETFE), and a perfluoroalkoxy fluorine resin (PFA), an olefin-based resin such as polyethylene and polypropylene or a mixture thereof, polyurethane, polyester, polyamide, a polyether nylon resin, a mixture of the olefin-based resin and an ethylene-vinyl acetate copolymer, and the like.

On the other hand, the liquid drug bag 26 includes a bag main body 96 having the storage space 26a (second internal space) that stores the liquid drug in advance, and a plurality of ports 98 provided on one side of the bag main body 96. The bag main body 96 is formed in a bag shape by overlapping sheets to seal the periphery.

Specifically, the bag main body 96 is formed in a substantially rectangular shape smaller than the bag main body 84 in plan view, and includes an accommodating portion 100 forming the storage space 26a and a seal part 102 (second seal part) which is a portion sealed around the accommodating portion 100. The bag main body 96 is made of the same material as the bag main body 84 of the PPP bag 24.

The seal part 102 includes one short side seal 102a of the bag main body 96, the other short side seal 102b of the bag main body 96, one long side seal 102c of the bag main body 96, and the other long side seal 102d of the bag main body 96 in plan view. In Fig. 3, the one short side seal 102a, the other short side seal 102b, the one long side seal 102c, and the other long side seal 102d are located on the lower side, the upper side, the left side, and the right side, respectively. In addition, two (one set of) bag holes 103 are provided at corner portions of the one and other long side seals 102c and 102d continuous with the one short side seal 102a.

The plurality of ports 98 are provided on the one short side seal 102a of the bag main body 96. The plurality of ports 98 include a connection port 104 to which the third tube 38 is connected and a filling port 106 to which a filling tube 108 is connected. The filling tube 108 is used when the liquid drug is stored in the storage space 26a, and is cut and sealed after being filled with the liquid drug. That is, the filling tube 108 is formed as short as possible with a sealed end portion in a state of the blood bag system 10 provided as a product. The plurality of ports 98 are welded in the state of communicating with the storage space 26a at the time of forming the seal part 102. The plurality of ports 98 are firmly fixed to end portions of the tubes 38 and 108 by an appropriate fixing means such as welding and adhesion.

The third tube 38 includes an elongated tube main body 39a forming most of its entire length, and a cylindrical end structure 39b that is continuous with an end portion of the tube main body 39a and is formed thick to be connected to the connection port 104. The above-described sealing member 42 is provided inside the end structure 39b. As the material forming the third tube 38, the materials listed for the second tube 36 may be applied.

Then, the blood bag system 10 includes a connection part 110 that connects the PPP bag 24 and the liquid drug bag 26, and forms a bag body 109 that can integrally handle the bags 24 and 26 at the time of setting the centrifugal transport device 12. Incidentally, the bag body 109 according to the present embodiment forms a part of the blood bag system 10, but is not limited thereto. The bag body 109 itself is a system of a medical bag having a function of storing a blood component, and thus, can also be referred to as a blood bag system.

The bag body 109 includes two sections (a first bag section 25 and a second bag section 27 which is a range including the liquid drug bag 26) with the connection part 110 as a base point. The first bag section 25 is a range including the PPP bag 24, but a form thereof is not limited to the illustrated example as long as the first bag section 25 has the storage space 24a and the seal part 90. Similarly, the second bag section 27 is the range including the liquid drug bag 26, but a form thereof is not limited to the illustrated example as long as the second bag section 27 has the storage space 26a and the seal part 102. For example, the bag body 109 may be provided as a planar body in which the first bag section 25 and the second bag section 27 are uniformly continuous.

The connection part 110 connects the one long side seal 90c (seal part 90 on the right side in Fig. 3) of the PPP bag 24 and the one long side seal 102c (seal part 102 on the left side in Fig. 3) of the liquid drug bag 26. That is, the one short side seal 90a to which the second tube 36 is connected and the one short side seal 102a to which the third tube 38 is connected are connected in the state of facing mutually opposite directions in the PPP bag 24 and the liquid drug bag 26. In the connection state, labels (not illustrated) attached to the PPP bag 24 and the liquid drug bag 26 face the same direction (inner side in the centrifugal direction).

More specifically, in the PPP bag 24, the connection part 110 is formed at a position (lower side of a portion inclined toward the one short side seal 90a) close to the one short side seal 90a in an extending portion of the one long side seal 90c. In addition, in the liquid drug bag 26, the connection part 110 is formed at a position close to the other short side seal 102b in an extending portion of the one long side seal 102c. A portion other than the connection part 110 between the one long side seals 90c and 102c is a gap 111 in which the one long side seal 90c and the one long side seal 102c are adjacent to each other in a non-contact manner.

The connection part 110 is configured as a connection sheet 112 that is flush with both the seal part 90 and the seal part 102. That is, the connection part 110 is formed by being molded integrally with the sheets forming the PPP bag 24 and the liquid drug bag 26 at the time of manufacturing the bags 24 and 26 and being sealed together with the formation of the seal parts 90 and 102. Incidentally, the connection sheet 112 may be configured separately from the PPP bag 24 and the liquid drug bag 26, and may be joined to each of the bags 24 and 26 after molding by welding, adhesion, or the like.

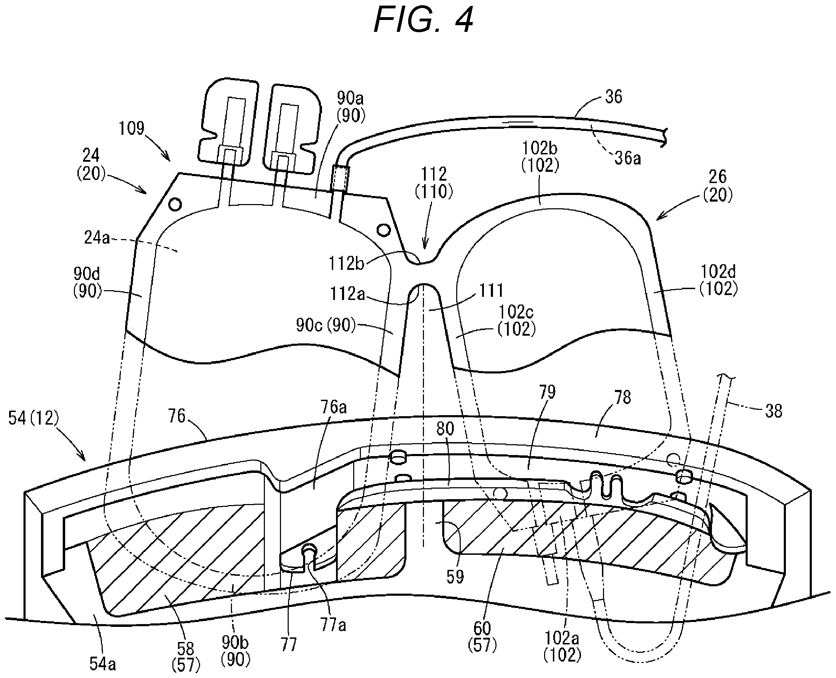

The connection sheet 112 continuously connects the PPP bag 24 and the liquid drug bag 26 not only at the time of being provided as a product but also when the PPP bag 24 and the liquid drug bag 26 are set in the centrifugal transport device 12 (see also Fig. 4). That is, in the set state, a lower edge 112a of the connection sheet 112 is caught by the boundary wall 59, and the centrifugal transport device 12 is operated in this state.

The connection sheet 112 (connection part 110) maintains the connection state in the set state of the centrifugal transport device 12, and is set to a strength capable of separating the PPP bag 24 and the liquid drug bag 26 under the operation of the user after the centrifugal transport device 12 is taken out. An extending length L along one of the long side seals 90c and 102c of the connection sheet 112 may have an appropriate length so as to obtain the above-described strength, and is set in, for example, a range of about 1 cm to 5 cm. In addition, a width W of the connection sheet 112 along a direction in which the PPP bag 24 and the liquid drug bag 26 are arrayed side by side may be appropriately set according to a width of the boundary wall 59 or the like, and is set in, for example, a range of 0.5 cm to 3 cm.

The connection sheet 112 may include a fragile portion 114 (see a dotted line in Fig. 3) that facilitates separation between the PPP bag 24 and the liquid drug bag 26. The fragile portion 114 can adopt, for example, a configuration in which a thickness of the connection sheet 112 itself is reduced, a configuration in which a cut line or a fold is formed, a configuration in which a cut is made in an upper edge 112b, or the like.

Although the liquid drug bag 26 and the third tube 38 are in a free state except for the connection portion on one end side in the present embodiment, it may be configured such that the other long side seal 102d and the third tube 38 are separably connected via a retention mechanism 116 (see a two-dot chain line in Fig. 3). For example, the retention mechanism 116 can be configured by fusing the third tube 38 and the substantially entire length of the other long side seal 102d in the extending direction with a predetermined resin material. A portion (an intermediate portion between one end and the other end) of the third tube 38 (tube main body 39a), which is curved and folded without kinking from the end structure 39b and extends linearly, is connected to the retention mechanism 116.

The blood bag system 10 according to the present embodiment is basically configured as described above, and operations thereof will be described hereinafter.

As described above, the blood bag system 10 stores the removed blood, obtained by removing a predetermined component (white blood cell) from the whole blood of the donor, in the blood bag 22 by using the pretreatment unit at the time of collecting blood by the user (medical worker). Thereafter, the user separates the separator unit 16 of the blood bag system 10 from the pretreatment unit and sets the separator unit 16 in the centrifugal transport device 12 in order to centrifuge the removed blood. The blood bag 22 is accommodated in the blood bag pocket 56 of the unit set area 54.

As illustrated in Fig. 2, the first to third tubes 34, 36, and 38 are disposed in a predetermined path on the upper surface 54a of the unit set area 54. Specifically, the second tube 36 is disposed so as to extend to the outer side in the centrifugal direction from the branching connector 32 in the left region 54a2 of the upper surface 54a and pass through the PPP clamp 70. Further, the second tube 36 is accommodated in the guide groove 79 of the tube retaining portion 76 on the radially outer side of the liquid drug bag pocket 60. On the other hand, the third tube 38 is also disposed so as to extend to the outer side in the centrifugal direction from the branching connector 32 in the left region 54a2 of the upper surface 54a and pass through the liquid drug clamp 72.

After the second and third tubes 36 and 38 are set, the user integrally sets the PPP bag 24 connected to the second tube 36 and the liquid drug bag 26 connected to the third tube 38 in the centrifugal transport device 12 as illustrated in Fig. 4. As described above, the PPP bag 24 and the liquid drug bag 26 are connected to each other via the connection part 110 (connection sheet 112).

Therefore, the other short side seal 90b of the PPP bag 24 at a position away from the connection part 110 and the one short side seal 102a of the liquid drug bag 26 can be separated in a direction of opening the gap 111 with the connection part 110 as the base point. Therefore, the user can direct the other short side seal 90b toward the PPP bag pocket 58 and can direct the one short side seal 102a toward the liquid drug bag pocket 60 in a state where the connection part 110 is right above the boundary wall 59.

Therefore, the PPP bag 24 is smoothly accommodated in the PPP bag pocket 58 from the other short side seal 90b, and the liquid drug bag 26 is also smoothly accommodated in the liquid drug bag pocket 60 from the one short side seal 102a. When being accommodated, the liquid drug bag 26 is accommodated in the liquid drug bag pocket 60 from the third tube 38 connected to the one short side seal 102a, so that the liquid drug bag pocket 60 accommodates a part of the third tube 38 to prevent the third tube 38 from projecting.

After the blood bag system 10 is set, the centrifugal transport device 12 centrifuges the removed blood in the blood bag 22 into blood components having different specific gravities (PPP, RBC, and the like) by rotating the centrifugal drum 48 under the control of the control unit 50. After such centrifugation, the centrifugal transport device 12 presses the blood bag 22 by the slider 82. As a result, PPP having a light specific gravity flows out from the blood bag 22, and the PPP flows through the first tube 34, the branching connector 32, and the second tube 36 in this order and flows into the PPP bag 24.

When PPP is transported from the blood bag 22 to the PPP bag 24, the centrifugal transport device 12 retracts the slider 82 so that the blood bag 22 can be taken out from the blood bag pocket 56. Thereafter, the user removes the blood bag system 10 from the centrifugal transport device 12. The PPP bag 24 and the liquid drug bag 26 are also taken out from the PPP bag pocket 58 and the liquid drug bag pocket 60 in the state of being integrated by the connection part 110.

After the take-out, the user performs an operation of separating the PPP bag 24 and the liquid drug bag 26. The connection part 110 (connection sheet 112) merely connects the small ranges of the seal part 90 of the PPP bag 24 and the seal part 102 of the liquid drug bag 26, and thus, is broken when the user applies a predetermined force or more in a direction of separating the PPP bag 24 and the liquid drug bag 26 from each other. A breaking line at this time is formed substantially along the extending direction of one of the long side seals 90c and 102c in the connection part 110. Therefore, the break line does not damage the accommodating portions 88 and 100 of the PPP bag 24 or the liquid drug bag 26.

After the PPP bag 24 and the liquid drug bag 26 are separated, the user suspends the liquid drug bag 26 on the stand to supply the red blood cell preservation solution in the liquid drug bag 26 to the blood bag 22. As a result, RBC containing the red blood cell preservation solution is stored in the blood bag 22.

Incidentally, the present invention is not limited to the above-described embodiment, and various modifications can be made in accordance with a gist of the invention. For example, the connection part 110 is formed in the sheet shape (connection sheet 112) in the present embodiment, but is not limited thereto, and can adopt various configurations. For example, the connection part 110 may be configured in a rod shape or a string shape.

Hereinafter, some other embodiments according to the present invention will be described. Incidentally, an element having the same configuration or the same function as that in the above-described embodiment will be denoted by the same reference sign, and the detailed description thereof will be omitted in the following description.

### [Second Embodiment]

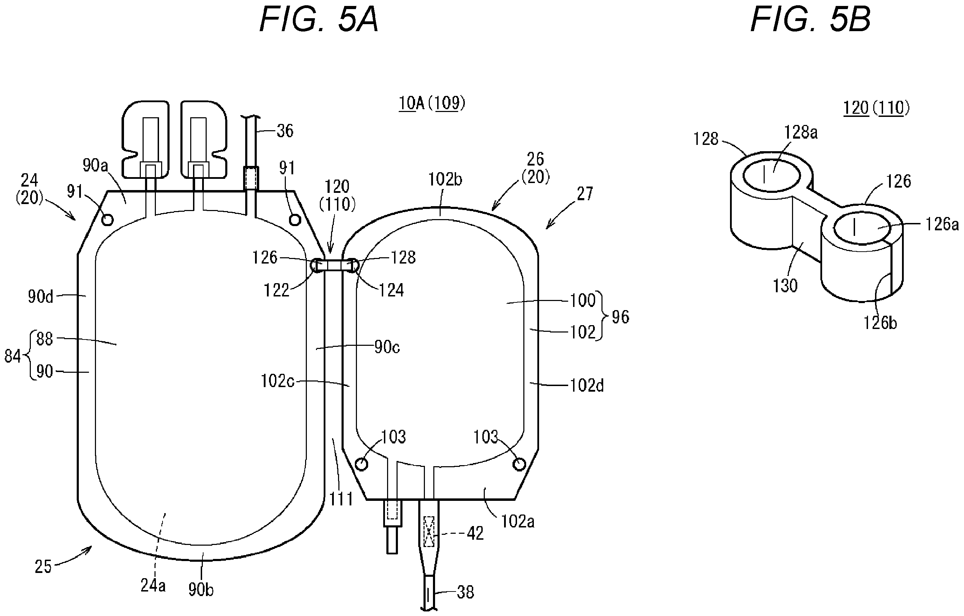

As illustrated in Fig. 5A, a blood bag system 10A according to a second embodiment is different from the blood bag system 10 described above in terms of using a connection member 120 for the connection part 110 between the PPP bag 24 and the liquid drug bag 26.

The connection member 120 passes through a PPP bag connection hole 122 (first hole) formed in the seal part 90 of the PPP bag 24 and passes through a liquid drug bag connection hole 124 (second hole) formed in the seal part 102 of the liquid drug bag 26. As illustrated in Fig. 5B, the connection member 120 includes a first part 126 connected to the PPP bag 24, a second part 128 connected to the liquid drug bag 26, and a relay part 130 connecting the first part 126 and the second part 128.

Further, the first part 126 is provided with a hole 126a and a slit 126b that communicates with the hole 126a and can pass through the seal part 90. On the other hand, the second part 128 is formed in an annular shape having a hole 128a, and is undetachably attached to the seal part 102.

The blood bag system 10A according to the second embodiment is configured as described above, and the PPP bag 24 and the liquid drug bag 26 can be separably connected by the connection member 120 (connection part 110). Therefore, the blood bag system 10A can obtain the same effects as those of the blood bag system 10 according to the first embodiment. In particular, the connection member 120 enables the PPP bag 24 and the liquid drug bag 26 to be connected again as necessary by freely attaching and detaching the PPP bag 24 and the liquid drug bag 26 to and from each other.

Incidentally, a structure of the connection member 120 is not particularly limited, and may be configured by, for example, one ring body capable of passing through both the PPP bag connection hole 122 and the liquid drug bag connection hole 124. In addition, the relay part 130 of the connection member 120 may be formed in various shapes (a block, a rod, a string, and the like along an upper end of the boundary wall 59).

### [Third Embodiment]

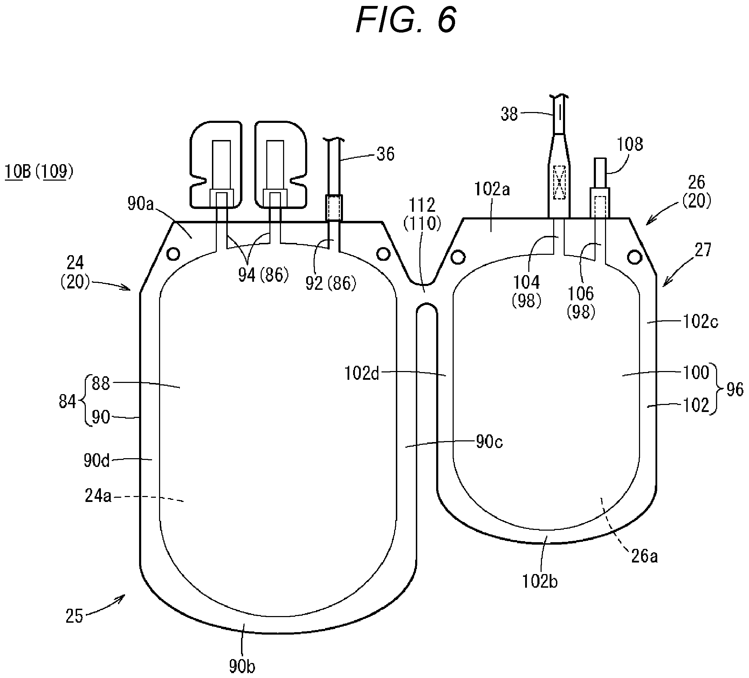

As illustrated in Fig. 6, a blood bag system 10B according to a third embodiment is different from the blood bag systems 10 and 10A in that the one short side seal 90a of the PPP bag 24 and the one short side seal 102a of the liquid drug bag 26 are connected in the state of facing the same direction. That is, the connection part 110 (connection sheet 112) connects the one long side seal 90c of the PPP bag 24 and the other long side seal 102d of the liquid drug bag 26.

Therefore, the second tube 36 extending from the one short side seal 90a of the PPP bag 24 and the third tube 38 extending from the one short side seal 102a of the liquid drug bag 26 protrude in the same direction. Therefore, when the liquid drug bag 26 is accommodated in the liquid drug bag pocket 60, the third tube 38 is immediately exposed from an opening of the liquid drug bag pocket 60.

Even in the configuration in which the third tube 38 is not accommodated in the liquid drug bag pocket 60 as described above, it is possible to prevent the third tube 38 from interfering with a peripheral structure of the centrifugal transport device 12 if the third tube 38 is shorter than that in the first embodiment.

Technical ideas and effects that can be grasped from the above-described embodiments are described as follows.

The blood bag system 10, 10A, or 10B includes the connection part 110 that separably connects the first seal part (seal part 90) and the second seal part (seal part 102), so that the first bag (PPP bag 24) and the second bag (liquid drug bag 26) can be integrally handled. That is, the user can smoothly perform the work of disposing the PPP bag 24 and the liquid drug bag 26 in the bag accommodating portion 57 of the centrifugal transport device 12 by collectively gripping the PPP bag 24 and the liquid drug bag 26. Further, the blood bag system 10, 10A, or 10B can separate the PPP bag 24 and the liquid drug bag 26 at the connection part 110 when the blood bag system 10, 10A, or 10B is taken out from the centrifugal transport device 12, and thus, the subsequent work is speeded up. Therefore, the blood bag systems 10, 10A, and 10B can significantly improve the work efficiency.

The bag accommodating portion 57 includes the first bag accommodating portion (PPP bag pocket 58) that accommodates the first bag (PPP bag 24) and the second bag accommodating portion (liquid drug bag pocket 60) accommodating the second bag (liquid drug bag 26), and the bag body 109 can accommodate the first and second bags in the connected state in the first and second bag accommodating portions, respectively. As a result, the bag body 109 can be easily set in the centrifugal transport device 12 with the PPP bag 24 and the liquid drug bag 26 being connected.

In addition, the connection part 110 is the connection sheet 112 in which the sheet forming the first bag (PPP bag 24) and the sheet forming the second bag (liquid drug bag 26) are continuous. As a result, the blood bag systems 10 and 10B can integrally mold the connection sheet 112 with the manufacture of the PPP bag 24 and the liquid drug bag 26, and the connection sheet 112 can be manufactured at low cost without increasing the number of steps.

In addition, the connection part 110 is the connection member 120 that passes through the first hole (PPP bag connection hole 122) formed in the first seal part (seal part 90) and passes through the second hole (liquid drug bag connection hole 124) formed in the second seal part (seal part 102) to perform connection, and is detachable from at least one of the first seal part and the second seal part. The blood bag system 10A can easily create the connection state between the PPP bag 24 and the liquid drug bag 26 by using the connection member 120. Further, the connection member 120 can connect the PPP bag 24 and the liquid drug bag 26 again as necessary even after the PPP bag 24 and the liquid drug bag 26 are once separated.

The connection part 110 connects a part on the back side in the insertion direction of the bag accommodating portion 57 on the long side (one long side seal 90c) of the first bag (PPP bag 24), formed in a rectangular shape, and a part on the back side in the insertion direction of the bag accommodating portion 57 on the long side (one long side seal 102c) of the second bag (liquid drug bag 26) formed in a rectangular shape. As a result, the blood bag systems 10, 10A, and 10B can hook the connection part 110 on the boundary wall 59 in a state where the PPP bag 24 is inserted into the PPP bag pocket 58 and the liquid drug bag 26 is inserted into the liquid drug bag pocket 60. Therefore, it is possible to favorably construct a suspended state of the PPP bag 24 in the PPP bag pocket 58 and a suspended state of the liquid drug bag 26 in the liquid drug bag pocket 60.

The connection part 110 connects the first seal part and the second seal part such that the first tubular body (second tube 36), which has one end connected to the first seal part (seal part 90) and extends from the first seal part, and the second tubular body (third tube 38), which has one end connected to the second seal part (seal part 102) and extends from the second seal part, extend in mutually opposite directions. As a result, the blood bag systems 10 and 10A can guide the third tube 38, formed to be long, to be inserted into the liquid drug bag pocket 60 together with the liquid drug bag 26, for example, at the time of setting the centrifugal transport device 12. Therefore, it is possible to easily form a state where the third tube 38 does not project from the centrifugal transport device 12.

In addition, the second tubular body (third tube 38) extends to a distal end side in the insertion direction of the bag accommodating portion 57, and the retention mechanism 116 that separably retains the second bag and the second tubular body is provided between the second bag and an intermediate portion between one end and the other end of the second tubular body. Since the third tube 38 is retained in the liquid drug bag pocket 60 by the retention mechanism 116, it is possible to suppress the third tube 38 from interfering with the insertion of the liquid drug bag 26 and to significantly improve the work efficiency at the time of setting.

In addition, the present invention also provides the blood bag system (bag body 109) molded into a bag shape by overlapping sheets, made of a resin material, and sealing the periphery thereof. The blood bag system includes: the first bag section 25 including the first internal space (storage space 24a) and the first seal part (seal part 90) that seals the periphery of the first internal space; the second bag section 27 including the second internal space (storage space 26a) and the second seal part (seal part 102) that seals the periphery of the second internal space; and the connection part 110 that separates the first bag section 25 and the second bag section 27 and connects the first bag section 25 and the second bag section 27 to be separable as an independent bag. As a result, the bag body 109 can integrally handle the first bag section 25 and the second bag section 27, and the work efficiency can be significantly improved.

## Claims

1. A blood bag system comprising
a bag body which includes a plurality of bags accommodated in a bag accommodating portion of a centrifugal transport device,
wherein the bag body includes:
a first bag including a first internal space and a first seal part that seals a periphery of the first internal space; and
a second bag including a second internal space and a second seal part that seals a periphery of the second internal space, and
a connection part that separably connects the first seal part and the second seal part is provided between the first seal part and the second seal part.

2. The blood bag system according to claim 1, wherein
the bag accommodating portion includes a first bag accommodating portion that accommodates the first bag and a second bag accommodating portion that accommodates the second bag, and
the bag body is capable of accommodating the first and second bags, which are in a connected state, respectively in the first and second bag accommodating portions.

3. The blood bag system according to claim 1 or 2, wherein
the connection part is a connection sheet in which a sheet forming the first bag and a sheet forming the second bag are continuous.

4. The blood bag system according to claim 1 or 2, wherein
the connection part is a connection member that passes through a first hole formed in the first seal part and a second hole formed in the second seal part to connect the first seal part and the second seal part, and is detachable from at least one of the first seal part and the second seal part.

5. The blood bag system according to any one of claims 1 to 4, wherein
the connection part connects a part of a back side in an insertion direction of the bag accommodating portion on a long side of the first bag, formed in a rectangular shape, and a part of a back side in the insertion direction of the bag accommodating portion on a long side of the second bag formed in a rectangular shape.

6. The blood bag system according to any one of claims 1 to 5, wherein
the connection part connects the first seal part and the second seal part such that a first tubular body, which has one end connected to the first seal part and extends from the first seal part, and a second tubular body, which has one end connected to the second seal part and extends from the second seal part, extend in mutually opposite directions.

7. The blood bag system according to claim 6, wherein
the second tubular body extends to a distal end side in the insertion direction of the bag accommodating portion, and
a retention mechanism that separably retains the second bag and the second tubular body is provided between the second bag and an intermediate portion between the one end and another end of the second tubular body.

8. A blood bag system, molded into a bag shape by overlapping sheets made of a resin material and sealing a periphery of the sheets, the blood bag system comprising:
a first bag section including a first internal space and a first seal part that seals a periphery of the first internal space;
a second bag section including a second internal space and a second seal part that seals a periphery of the second internal space; and
a connection part that separates the first bag section and the second bag section and connects the first bag section and the second bag section to be separable as an independent bag.
